(19) Europäisches Patentamt
European Patent Office
Office européen des brevets

(11) **EP 4 628 160 A1**

(12) **EUROPEAN PATENT APPLICATION**
published in accordance with Art. 153(4) EPC

(43) Date of publication:
**08.10.2025 Bulletin 2025/41**

(21) Application number: **23897542.9**

(22) Date of filing: **17.11.2023**

(51) International Patent Classification (IPC):
***A61N 5/10*** [(2006.01)]

(52) Cooperative Patent Classification (CPC):
**A61N 5/10**

(86) International application number:
**PCT/JP2023/041401**

(87) International publication number:
**WO 2024/116891 (06.06.2024 Gazette 2024/23)**

(84) Designated Contracting States:
**AL AT BE BG CH CY CZ DE DK EE ES FI FR GB
GR HR HU IE IS IT LI LT LU LV MC ME MK MT NL
NO PL PT RO RS SE SI SK SM TR**
Designated Extension States:
**BA**
Designated Validation States:
**KH MA MD TN**

(30) Priority: **28.11.2022 JP 2022189539**

(71) Applicant: **The University of Osaka
Suita-shi, Osaka 565-0871 (JP)**

(72) Inventors:
• **YAGI, Masashi
Suita-shi, Osaka 565-0871 (JP)**
• **MINAMI, Kazumasa
Suita-shi, Osaka 565-0871 (JP)**
• **SHIMIZU, Shinichi
Suita-shi, Osaka 565-0871 (JP)**

(74) Representative: **Plasseraud IP
104 Rue de Richelieu
CS92104
75080 Paris Cedex 02 (FR)**

(54) **DOSE EVALUATION METHOD, IRRADIATION PLANNING METHOD, DOSE EVALUATION DEVICE, CONTROL PROGRAM, AND RECORDING MEDIA**

(57) A clinical dose is evaluated accurately. A dose evaluating method includes: a calculation information obtaining step (S 1) of obtaining calculation target information including (i) information that pertains to radiation with which an irradiation target is irradiated and that includes a dose or a dose rate of the radiation and (ii) information that pertains to types of cells constituting the irradiation target which is irradiated with the radiation; an absorbed dose obtaining step (S2) of obtaining, for each of the types of the cells constituting the irradiation target, an absorbed dose that indicates energy of the radiation absorbed by cells of the each of the types; and a calculating step (S4) of calculating, for each of the types of the cells, a clinical dose of the radiation with which the irradiation target is irradiated, on the basis of the calculation target information and the absorbed dose information.

FIG. 2

**Description**

Technical Field

The present invention relates to a dose evaluating method of evaluating a clinical dose in radiation irradiation, an irradiation planning method, a dose evaluating device, a control program, and a recording medium.

Background Art

Conventionally, in a case where radiotherapy is performed, a dose distribution in a body is estimated in order to predict an effect on an irradiation target such as a tumor formed in an organ and a normal organ neighboring the tumor, and then a treatment plan is created. For example, a clinical dose is calculated by the method disclosed in Patent Literature 1 or 2, a dose distribution in the body of a target is estimated on the basis of the clinical dose, and a treatment plan is created on the basis of the dose distribution.

Citation List

[Patent Literature]

[Patent Literature 1]
Japanese Patent No. 5454989
[Patent Literature 2]
Japanese Patent Application Publication Tokukai No. 2019-180908

Summary of Invention

Technical Problem

A conventional clinical dose estimating method has room for improvement in accuracy of a result of estimation of a clinical dose.

Solution to Problem

In order to attain the above object, a dose evaluating method in accordance with a first aspect of the present invention includes: a calculation information obtaining step of obtaining calculation target information including (i) information that pertains to radiation with which an irradiation target is irradiated and that includes a dose or a dose rate of the radiation and (ii) information that pertains to types of cells constituting the irradiation target which is irradiated with the radiation; an absorbed dose obtaining step of obtaining absorbed dose information that indicates, for each of the types of the cells constituting the irradiation target, an absorbed dose which is energy of the radiation absorbed by cells of the each of the types; and a calculating step of calculating, for each of the types of the cells, a clinical dose of the radiation with which the irradiation target is irradiated, on the basis of the calculation target information and the absorbed dose information.

An irradiation planning method in accordance with a second aspect of the present invention is a method of creating a plan of irradiation of an irradiation target with radiation, on the basis of a clinical dose calculated by the dose evaluating method described in the first aspect.

A dose evaluating device in accordance with a third aspect of the present invention includes: a calculation information obtaining section which obtains calculation target information including (i) information that pertains to radiation with which an irradiation target is irradiated and that includes a dose or a dose rate of the radiation and (ii) information that pertains to types of cells constituting the irradiation target which is irradiated with the radiation; an absorbed dose obtaining section which obtains absorbed dose information that indicates, for each of the types of the cells constituting the irradiation target, an absorbed dose which is energy of the radiation absorbed by cells of the each of the types; and a calculating section which calculates, for each of the types of the cells, a clinical dose of the radiation with which the irradiation target is irradiated, on the basis of the calculation target information and the absorbed dose information.

The dose evaluating device in accordance with each aspect of the present invention may be realized by a computer. In this case, the present invention also encompasses, in its scope, (i) a control program for a dose evaluating device, the control program causing the dose evaluating device to be realized by a computer, by causing the computer to operate as each section (software element) included in the dose evaluating device and (ii) a computer-readable recording medium in which the control program is recorded.

Advantageous Effects of Invention

The dose evaluating method etc. of the present invention make it possible to more accurately evaluate a clinical dose than a conventional method.

Brief Description of Drawings

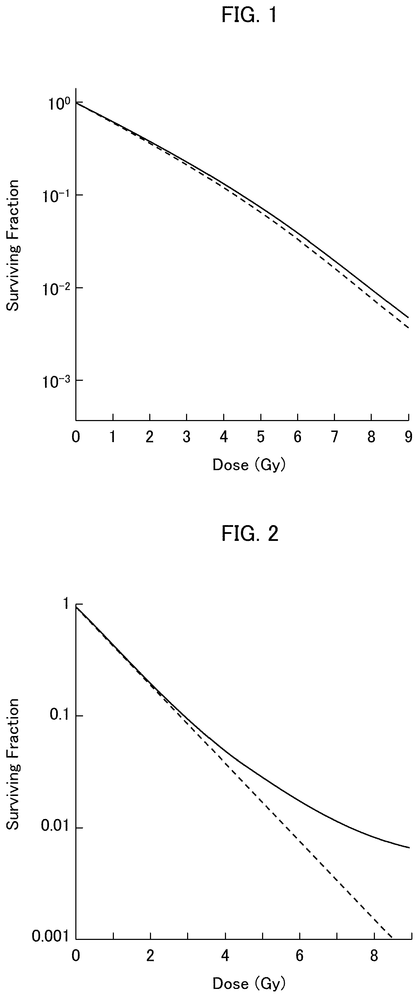

Fig. 1 is an example of a graph showing the relationship between (i) a surviving fraction of human submandibular gland-derived adenocarcinoma cells (HSG cells) and (ii) the dose of radiation with which the cells have been irradiated, in an experiment of irradiation with a heavy particle beam at an ultrahigh dose rate.
Fig. 2 is an example of a graph showing the relationship between (i) a surviving fraction of bronchus-derived epithelial cells (Nuli-1 cells) and (ii) the dose of radiation with which the cells have been irradiated, in an experiment of irradiation with a heavy particle beam at an ultrahigh dose rate.

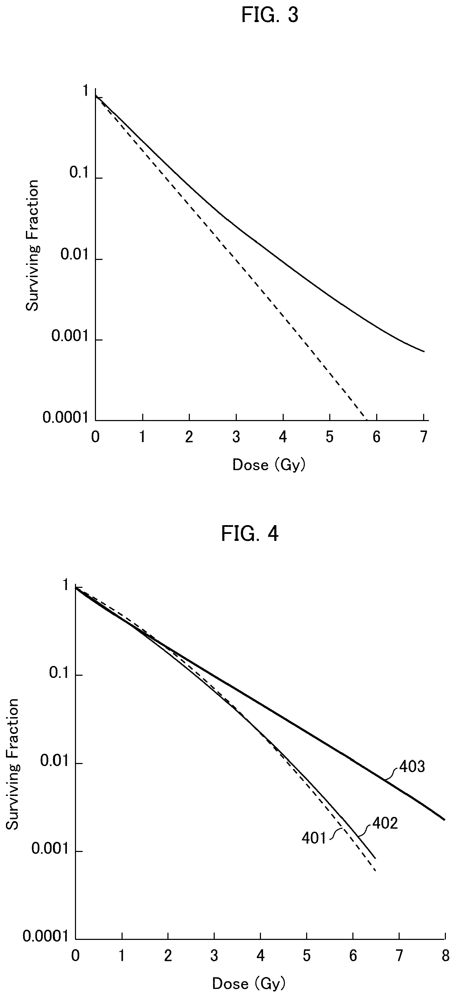

Fig. 3 is an example of a graph showing the relationship between (i) a surviving fraction of human skin fibroblasts (HDF cells) and (ii) the dose of radiation with which the cells have been irradiated, in an experiment of irradiation with a heavy particle beam at an ultrahigh dose rate.
Fig. 4 is an example of a graph showing the relationship between (i) a surviving fraction of the other type of cells X differing from the HSG cells, the Nuli-1 cells, and the HDF cells and (ii) the dose of radiation with which the cells have been irradiated, in an experiment of irradiation with a heavy particle beam at an ultrahigh dose rate.

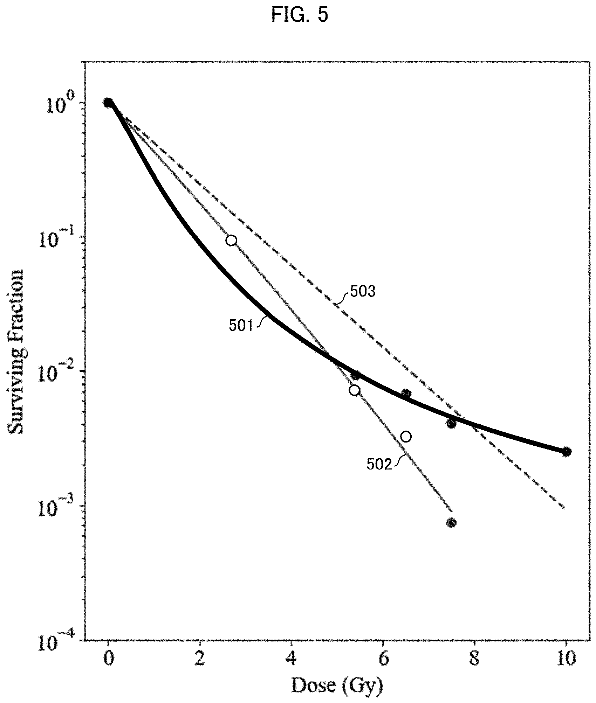

Fig. 5 shows (i) measurement results obtained, regarding the dose of a heavy particle beam with which cells are irradiated and the surviving fraction of the cells, by an experiment of irradiation with the heavy particle beam and (ii) an example of a graph obtained by carrying out calculation in accordance with the present invention so that the graph well matches the measurement results.

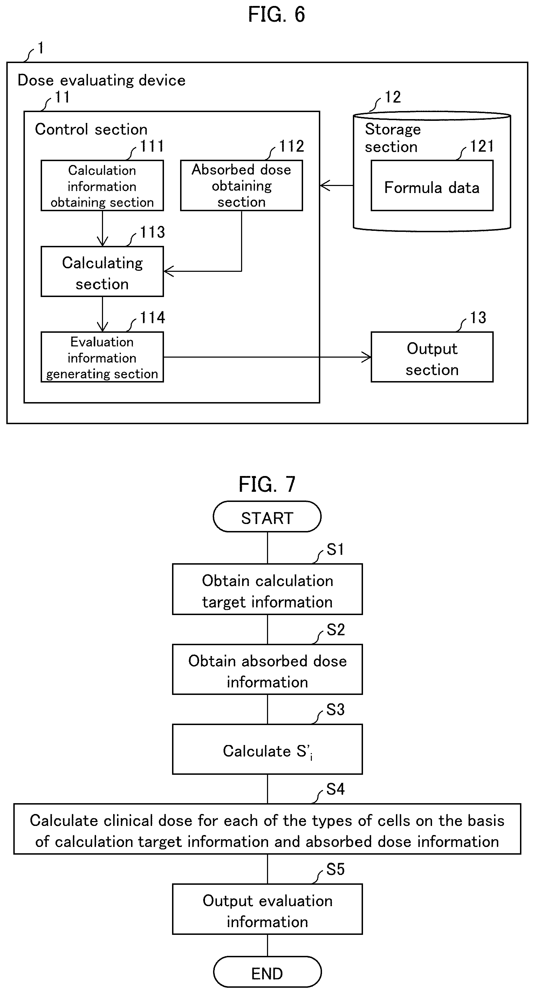

Fig. 6 is a block diagram illustrating an example of the configuration of a dose evaluating device.
Fig. 7 is a flowchart illustrating an example of a flow of processes carried out in the dose evaluating device.

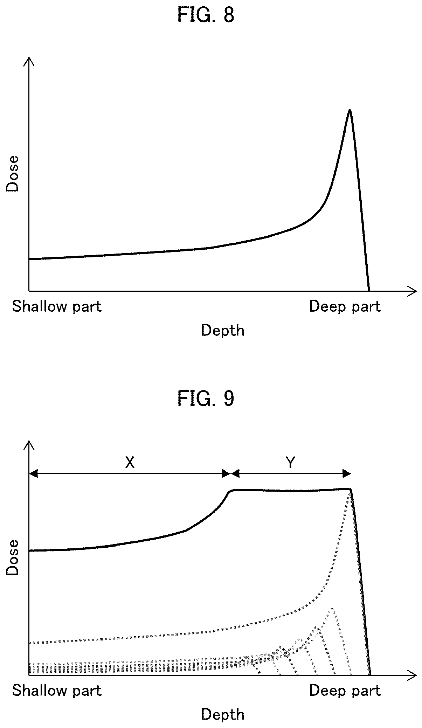

Fig. 8 is a graph showing an example of the relationship between (i) an absorbed dose that indicates the amount of the energy of radiation absorbed by an irradiation target in a case where the irradiation target is irradiated with the radiation and (ii) a depth in the irradiation target from a body surface.
Fig. 9 is a graph showing the relationship between (i) an absorbed dose that indicates the amount of the energy of radiation absorbed by an irradiation target in a case where the irradiation target is irradiated with superposed beams of a plurality of energies in radiotherapy and (ii) a depth in the irradiation target from a body surface.

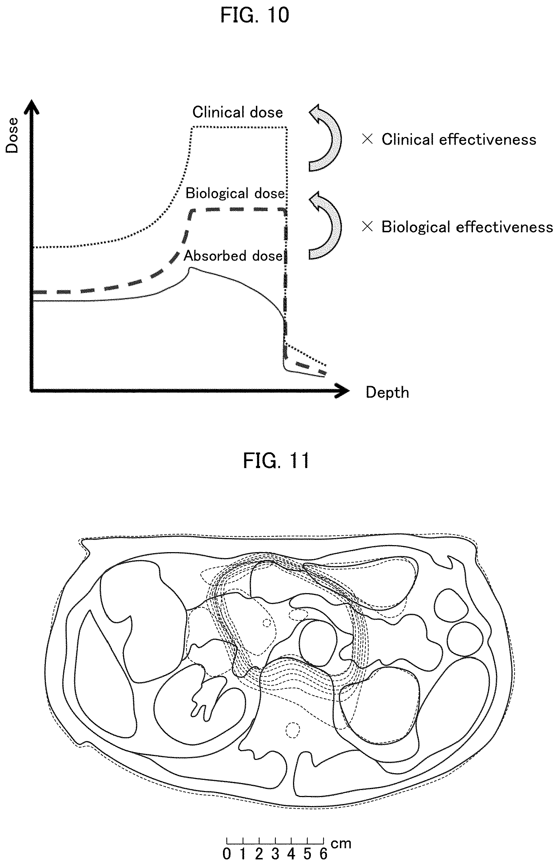

Fig. 10 is a relationship graph showing the relationship between an absorbed dose, a biological dose, and a clinical dose.
Fig. 11 is an example of a dose distribution map showing a result of estimating a dose distribution in heavy particle radiotherapy with use of a conventional dose distribution calculating method.

Description of Embodiments

Embodiment 1

<Heavy particle radiotherapy>

A dose evaluating method, an irradiation planning method, etc. in accordance with the present invention can be applied to heavy particle radiotherapy. The heavy particle radiotherapy is, among various types of radiotherapy which are some of cancer therapies, radiotherapy in which a heavy particle beam (for example, a carbon ion beam) is used. Heavy particle beams have a higher ability to damage and kill cancer cells in a tumor region than radiation that has been used in conventional radiotherapy (for example, a photon beam (such as an X-ray), an electron beam, or a proton beam). Therefore, the heavy particle radiotherapy is radiotherapy suitable to treat cancer.
Fig. 8 is a graph showing an example of the relationship between (i) an absorbed dose that indicates the amount of the energy of radiation absorbed by an irradiation target in a case where the irradiation target is irradiated with the radiation and (ii) a depth in the irradiation target from a body surface. As an example, Fig. 8 shows the relationship between (i) an absorbed dose of a proton beam, which is one of particle beams, and (ii) the depth in an irradiation target from a body surface. As shown in Fig. 8, some radiation has the property of, in a case where a human body that is an irradiation target is irradiated therewith, exhibiting a full effect in a deep part of the human body or the like. Particle beams such as a carbon beam have this property.

Fig. 9 is a graph showing the relationship between (i) an absorbed dose that indicates the amount of the energy of radiation absorbed by an irradiation target in a case where the irradiation target is irradiated with superposed beams of a plurality of energies in radiotherapy and (ii) a depth in the irradiation target from a body surface. As shown in Fig. 9, in radiotherapy, a method of irradiating a tumor region (a region indicated by an arrow Y in Fig. 9) with superposed beams of a plurality of energies is employed.

Particle beams have the property of, in a case where an irradiation target such as a human body is irradiated therewith, exhibiting a full effect in a deep part of the body of the irradiation target. Use of this property makes it possible to intensively irradiate a tumor region with radiation and makes it unlikely to damage a region of normal cells (a region indicated by an arrow X in Fig. 9) that neighbors the tumor region, by irradiating the tumor region with superposed beams of a plurality of energies in particle radiotherapy. Thus, the heavy particle radiotherapy can result in a shorter treatment duration than the conventional radiotherapy, and can realize a higher quality of life (QOL) for a patient who receives the radiotherapy. In the heavy particle radiotherapy, a dose distribution of a heavy particle beam in a body is estimated in advance. On the basis of a result of the estimation of the dose distribution, the dose of the radiation with which a tumor is irradiated during treatment is determined, and a treatment plan is formulated.

<Conventional method of estimating dose distribution in heavy particle radiotherapy>

An absorbed dose is the amount of energy absorbed by a unit mass of a substance upon irradiation with radiation. It is known that the degree of the effect of heavy particles, such as carbon particles, on cells irradiated with the heavy particles cannot be evaluated only by an absorbed dose, and it is impossible to estimate a dose distribution in heavy particle radiotherapy only on the basis of a physical absorbed dose (also referred to as a physical dose) as in general photon radiotherapy and general proton radiotherapy. In order to estimate the dose distribution, a biological dose that is a dose in which biological effectiveness is taken into consideration and a clinical dose that is a dose in which clinical effectiveness is taken into consideration must be taken into consideration.

Fig. 10 shows the relationship between an absorbed dose, a biological dose, and a clinical dose. The biological dose is a value obtained by multiplying the absorbed dose by the value of relative biological effectiveness (RBE). The RBE is an indicator used to indicate that the strength of a biological effect varies depending on the type of radiation and a depth. The RBE is a ratio between (i) the absorbed dose of radiation (reference X-ray) which absorbed dose is necessary to obtain a certain effect and (ii) the absorbed dose of radiation (radiation with which an irradiation target is irradiated) which absorbed dose is necessary to obtain the same effect. The value of the RBE varies depending on, for example, the type of radiation. The RBE is a value obtained by carrying out, in advance, an experiment of irradiation of a cultured cell strain with a heavy particle beam (cell irradiation experiment) and a biological evaluation by the cell irradiation experiment.

The clinical dose is the dose of radiation with which an irradiation target such as a tumor is actually irradiated, and is typically obtained by multiplying the calculated biological dose by an empirical coefficient (for example, 1.46).

The value of the RBE that should be applied is experimentally determined in accordance with, for example, the type and the quality of radiation. Therefore, in estimation of a dose distribution in heavy particle radiotherapy, a biological dose is calculated with use of the RBE that should be applied to an absorbed dose, and the dose (clinical dose) of radiation with which a tumor is irradiated during treatment is further determined from the calculated biological dose.

In a case where a treatment plan of heavy particle radiotherapy is created, dose distribution calculation is carried out on the basis of doses (biological dose, clinical dose) in each of which biological effectiveness is taken into consideration, in addition to an absorbed dose. Then, a dose distribution is estimated which indicates the distribution of energy imparted to an irradiation target such as a tumor in a case where the irradiation target is irradiated with a heavy particle beam in accordance with the calculated clinical dose. Fig. 11 is an example of a dose distribution map showing a result of estimating a dose distribution in heavy particle radiotherapy with use of a conventional dose distribution calculating method (described later). As illustrated in Fig. 11, a dose distribution map is created by drawing, on a medical image (in Fig. 11, parts indicated by solid lines) of an irradiation target, contour lines each obtained by connecting, with a line (broken line in Fig. 11), positions at which the same dose has been estimated.

<Conventional method of calculating biological dose in heavy particle radiotherapy>

A conventional method of calculating a biological dose can be, for example, the method disclosed in Japanese Patent Application Publication Tokukai No. 2019-180908. An example of the conventional method of calculating a biological dose is described below. Since a graph that is obtained in an experiment of irradiation of a cultured cell strain with radiation (cell irradiation experiment) and that shows the relationship between a dose and a surviving fraction matches a model indicated by formula (1) below, a method of calculating a cell surviving fraction $S_i$ by formula (1) below is often used.

$$S_i = exp\left(-\alpha_i d_i - \beta_i d_i^2\right) \qquad \cdots \text{formula (1)}$$

Note, here, that $d_i$ is an absorbed dose in standard cells. In estimation of a clinical dose in heavy particle radiotherapy, a human submandibular gland-derived adenocarcinoma cell strain (HSG cells) is often selected as the standard cells.

The coefficients $\alpha$ and $\beta$ are each a value based on a function in which linear energy transfer (LET) is a variable and which is created from a survival curve obtained from an experiment of irradiation of the cells with radiation.

$$\alpha = f(LET)$$

$$\beta = g(LET)$$

On the basis of the cell surviving fraction $S_i$ calculated by formula (1), a biological dose $d_{bio,i}$ at a focus position i is calculated by formula (2) below that is a conventional estimation formula. On the basis of the biological dose, a clinical dose is calculated.

$$d_{bio,i} = \frac{\sqrt{\alpha_X^2 - 4\beta_X \ln(S_i)} - \alpha_X}{2\beta_X} \qquad \cdots \text{formula (2)}$$

<Novel findings from irradiation with heavy particle beam at ultrahigh dose rate>

In radiotherapy in which a photon beam (for example, an X-ray), an electron beam, or a proton beam is used, irradiation may be carried out at a dose rate that is much higher than a typical dose rate (for example, not less than 400 times higher than a dose rate used in typical radiotherapy). It is known that, in a case where irradiation with radiation is carried out at this ultrahigh dose rate, the effect of preventing damage to a normal tissue while maintaining a local control rate with respect to a tumor is seen. This effect is referred to as a FLASH effect. Note that a dose indicates the intensity of radiation, whereas a "dose rate" means the dose of radiation with which a certain region is irradiated per unit time. A "dose rate used in typical radiotherapy" (hereinafter may be referred to as "typical dose rate") means, for example, a dose rate of 0.03 Gy/second (in a case where an irradiation volume of 1 liter (10 cm $\times$ 10 cm $\times$ 10 cm) is irradiated with radiation of a physical dose of 2 Gy with a range of 20g/cm$^2$). An "ultrahigh dose rate" means, for example, a dose rate of not less than 40 Gy/second.

Although the FLASH effect in a case where irradiation with a photon beam (for example, an X-ray), an electron beam, or a proton beam is carried out has been reported, the FLASH effect in a case where irradiation with a heavy particle beam is carried out has not been reported. In addition, under the present circumstances, there are a small number of facilities in which heavy particle radiotherapy can be carried out, and it is not easy to carry out an experiment of irradiation with a heavy particle beam. In particular, there are an extremely limited number of heavy particle beam irradiating devices that can carry out irradiation at ultrahigh dose rates. Therefore, no academic determination has been made regarding the FLASH effect of a heavy particle beam.

The inventors of the present invention conducted diligent studies, and, as a result, clearly confirmed the FLASH effect in a case where irradiation with a heavy particle beam is carried out at an ultrahigh dose rate. Figs. 1 to 4 are each an example of a graph showing the relationship between (i) a surviving fraction of cells which are an irradiation target and (ii) the dose of radiation with which the cells have been irradiated, in an experiment of irradiation with a heavy particle beam at an ultrahigh dose rate. In each of Figs. 1 to 4, cells which are an irradiation target are as follows.

· Fig. 1: Human submandibular gland-derived adenocarcinoma cells (HSG cells)

· Fig. 2: Bronchus-derived epithelial cells (Nuli-1 cells)

· Fig. 3: Human skin fibroblasts (HDF cells)

· Fig. 4: The other type of cells X differing from the HSG cells, the Nuli-1 cells, and the HDF cells

Fig. 1 is a graph showing results of irradiating the HSG cells, which are standard cells, with a heavy particle beam at a typical dose rate and an ultrahigh dose rate. In Fig. 1, a broken line is a survival curve showing the relationship between a surviving fraction and the dose of the heavy particle beam with which irradiation was carried out, in a case where the irradiation with the heavy particle beam was carried out at the typical dose rate, whereas a solid line is a survival curve showing the relationship between a surviving fraction and the dose of the heavy particle beam with which irradiation was carried out, in a case where the irradiation with the heavy particle beam was carried out at the ultrahigh dose rate. Fig. 2 is a graph showing results of irradiating the Nuli-1 cells with a heavy particle beam at a typical dose rate and an ultrahigh dose rate. Fig. 3 is a graph showing results of irradiating the HDF cells with a heavy particle beam at a typical dose rate and an ultrahigh dose rate. Fig. 2 shows survival curves in a case where the Nuli-1 cells were irradiated with the heavy particle beam at the typical dose rate and the ultrahigh dose rate. Fig. 3 shows survival curves in a case where the HDF cells were irradiated with the heavy particle beam at the typical dose rate and the ultrahigh dose rate. In each of Figs. 2 and 3, a broken line is a survival curve showing the relationship between a surviving fraction and the dose of the heavy particle beam with which irradiation was carried out, in a case where the irradiation with the heavy particle beam was carried out at the typical dose rate, whereas a solid line is a survival curve showing the relationship between a surviving fraction and the dose of the heavy particle beam with which irradiation was carried out, in a case where the irradiation with the heavy particle beam was carried out at the ultrahigh dose rate.

As shown in Figs. 2 and 3, in a case where cells are irradiated with radiation at a typical dose rate (broken lines in the graphs), the surviving fraction of the cells decreases with increasing dose. In contrast, in a case where the cells are irradiated with the radiation at a high dose rate (solid lines in the graphs), the surviving fraction of the cells may be higher than that in a case where the cells are irradiated with the radiation at the typical dose rate (FLASH effect).

The inventors of the present invention conducted detailed studies on the FLASH effect in a case where irradiation with a heavy particle beam is carried out at an ultrahigh dose rate. As a result, it was clearly confirmed, as shown in Figs. 2 and 3, that, in a case where cells are irradiated with a heavy particle beam, there is a difference in surviving fraction of the cells between a typical dose rate and an ultrahigh dose rate, in a high dose range. Specifically, irradiation at an ultrahigh dose rate resulted in a higher surviving fraction than irradiation at a typical dose rate. Then, it was found that a degree by which the surviving fraction at the time of the irradiation at the ultrahigh dose rate was higher than that at the time of the irradiation at the typical dose rate became larger with increasing dose.

Moreover, when a plurality of types of cells were irradiated at the same ultrahigh dose rate, it was revealed that a response (surviving fraction) to a heavy particle beam varies for each of the types of cells. That is, it was found that a survival curve of HSG cells (graph in Fig. 1) does not match survival curves of the other types of cells differing from the HSG cells (graphs in Figs. 2 and 3) and that a survival curve is likely to vary for each of the types of cells. Fig. 4 shows survival curves each showing the relationship between a surviving fraction and the dose of a heavy particle beam with which irradiation was carried out, in a case where the other type of cells X differing from the HSG cells, the Nuli-1 cells, and the HDF cells were irradiated with the heavy particle beam at a typical dose rate or an ultrahigh dose rate. Also regarding the other type of cells X differing from the HSG cells, the Nuli-1 cells, and the HDF cells, it was suggested, as shown by the graph in Fig. 4, that a survival curve at a high dose rate becomes like not a thin solid line (reference sign 402) in the graph which thin solid line is a curve analogous to the survival curve at the typical dose rate (thin broken line in the graph: reference sign 401), but a bold solid line (reference sign 403) in the graph which bold solid line greatly differs from the thin solid line. Note that, in Fig. 4, the graph shown by the broken line and the graphs shown by the solid lines are graphs obtained on the basis of the conventional methods of calculating a biological dose and a cell surviving fraction.

Furthermore, from the fact that, in a case where the irradiation with the heavy particle beam was carried out, a phenomenon in which a response (surviving fraction) varied depending on the type of the cells was observed, it was confirmed that the surviving fraction of cells which are irradiated with radiation is also affected by (i) the type of the radiation with which irradiation is carried out, such as a heavy particle beam, a photon beam, an electron beam, or a proton beam, and (ii) the quality of the radiation which quality indicates energy imparted to an irradiation target in a case where irradiation with the radiation is carried out.

As has been described, by studying the FLASH effect in a case where irradiation with a heavy particle beam is carried out at an ultrahigh dose rate, it was found that the surviving fraction of cells which are irradiated with radiation depends on

· the type of the cells (cell),
· the type of the radiation (ion), and
· the quality of the radiation (energy).

Furthermore, it was found that the surviving fraction of the cells which are irradiated with the radiation also depends on

· the dose of the radiation (dose) or
· the dose rate of the radiation (dose_rate)

which dose rate is obtained by converting, into a dose per unit time, the dose of the radiation with which a certain region is irradiated.

Note, here, that the type of cells indicates that, for example, the position of the cells in the body of an irradiation target and the role of the cells vary depending on the type. For example, "different types of cells" means cells constituting different organs etc. (for example, liver, skin) of a target. Specific examples of the type of cells include cancer cells of lung cancer, liver cancer, and pancreatic cancer that are often subjected to radiotherapy. In order that the degree of an effect on normal cells is evaluated, the specific examples of the type of cells also include normal cells of skin, an intestine, and a lung.

<Method of calculating biological dose in accordance with the present invention>

In the conventional estimating method, regarding a dose distribution in a body, a surviving fraction $S_i$ is calculated by applying an absorbed dose in HSG cells, which are standard cells, to formula (1), and then a biological dose $d_{bio,i}$ is estimated by substituting the surviving fraction $S_i$ into formula (2), regardless of the type of cells which are an irradiation target.

As has been described, it was found that, in a case where these conventionally used estimation formulas are applied to calculation of a dose distribution of a heavy particle beam at an ultrahigh dose rate, the value of a surviving fraction calculated may differ from the value of an actual surviving fraction.

On the basis of these newly obtained findings, the inventors of the present invention found that formula (3) below, which has been obtained by improving formula (1) above, matches an actual surviving fraction.

$$S'_i = exp\left(-\alpha_{new}d_i - \beta_{new}d_i^2\right) + C \quad \cdots \text{formula (3)}$$

Note, here, that $d_i$ is an absorbed dose for each of the types of cells, and the coefficient $a_{new}$, the coefficient $\beta_{new}$, and the correction term C are as follows.

$$\alpha_{new} = f(cell, ion, energy)$$

$$\beta_{new} = g(cell, ion, energy)$$

$$C = h(cell, ion, energy, dose, dose\_rate)$$

As has been described, the surviving fraction of cells which are irradiated with radiation depends on the type of the cells (cell), the type of the radiation (ion), and the quality of the radiation (energy), and also depends on the dose of the radiation (dose) or the dose rate of the radiation (dose_rate). Formula (3) above expresses these findings as a relationship formula.

The coefficient $a_{new}$, the coefficient $\beta_{new}$, and the correction term C allows calculation of, from formula (3), a value indicating a surviving fraction $S'_i$ that depends on the type of cells, the type of radiation, the quality of the radiation, and the dose (or the dose rate) of the radiation.

The coefficients $\alpha_{new}$ and $\beta_{new}$ are each a value determined by carrying out an experiment of irradiating a cultured cell strain (cells of a type for which a dose is to be calculated) with radiation (cell irradiation experiment), as in determining, in formula (1) in the conventional method, the coefficients $\alpha$ and $\beta$ with respect to standard cells. By carrying out the cell irradiation experiment, a survival curve indicating the relationship between a dose and a surviving fraction in the cells is obtained. Then, the values of the coefficients $\alpha_{new}$ and $\beta_{new}$ are determined so that this data well matches a result calculated on the basis of formula (3). The correction term C is a value for correcting a surviving fraction obtained with use of the coefficients $\alpha_{new}$ and $\beta_{new}$ so that the surviving fraction conforms to a result of an experiment of irradiation at an ultrahigh dose rate. By correcting, with the correction term C, a surviving fraction obtained with use of the coefficients $\alpha_{new}$ and $\beta_{new}$, it is possible to calculate a value that indicates a more accurate surviving fraction $S'_i$ in which a difference in surviving fraction between a typical dose rate and an ultrahigh dose rate and a difference in surviving fraction according to the type of cells are reflected.

Moreover, the inventors of the present invention found that formula (4) below matches an actual surviving fraction more than formula (3).

$$S'_i = exp\left(-\alpha_{new}d_i - \beta_{new}d_i^2 \cfrac{1}{1 + \cfrac{\beta_{new}}{\gamma}d_i + \delta d_i^2}\right) \cdots \text{formula (4)}$$

Note, here, that, as in formula (3), $d_i$ is an absorbed dose for each of the types of cells. The coefficient $\alpha_{new}$, the coefficient $\beta_{new}$, the coefficient y, and the coefficient $\delta$ are as follows.

$$\alpha_{new} = f(cell, ion, energy)$$

$$\beta_{new} = g(cell, ion, energy)$$

$$\gamma = h(cell, ion, energy, dose, dose\_rate)$$

$$\delta = i(cell, ion, energy, dose, dose\_rate)$$

The point where formula (4) is derived from the findings that the surviving fraction of cells which are irradiated with radiation depends on the type of the cells (cell), the type of the radiation (ion), and the quality of the radiation (energy) and also depends on the dose of the radiation (dose) or the dose rate of the radiation (dose_rate) is the same as that in a case where formula (3) is derived. By formula (4) including the coefficients $\alpha_{new}$, $\beta_{new}$, $\gamma$, and $\delta$, a value indicating a surviving fraction $S'_i$ that depends on the type of cells, the type of radiation, the quality of the radiation, and the dose (or the dose rate) of the radiation is calculated.

By carrying out a cell irradiation experiment, a survival curve indicating the relationship between a dose and a surviving fraction in cells is obtained. Then, the values of the coefficients $\alpha_{new}$, $\beta_{new}$, $\gamma$, and $\delta$ are determined so that this data well matches a result calculated on the basis of formula (4). By formula (4), it is possible to calculate a value that indicates a more accurate surviving fraction $S'_i$ in which a difference in surviving fraction between a typical dose rate and an ultrahigh dose rate and a difference in surviving fraction according to the type of cells are reflected. Moreover, formula (4) is excellent in that it is possible to accurately calculate a surviving fraction in a high dose range.

On the basis of formula (5) below, in which the surviving fraction $S'_i$ calculated with use of formula (3) or (4) is applied to the conventional formula (2), it is possible to calculate a biological dose $d_{bio,i}$ at a focus position i. Unlike the conventional formula (2), in formula (5), calculation is carried out with use of the surviving fraction $S'_i$ which has been calculated for each of the types of cells by formula (3) or (4).

$$d_{bio,i} = \frac{\sqrt{\alpha_X^2 - 4\beta_X \ln(S'_i)} - \alpha_X}{2\beta_X} \quad \cdots \text{formula (5)}$$

Furthermore, it is possible to calculate a clinical dose as follows. That is, the biological dose $d_{bio,i}$ calculated with use of formula (5) is rescaled by multiplying the biological dose $d_{bio,i}$ by an empirical coefficient (as has been described, 1.46 is often used) for converting a cell response to a tissue response. The clinical dose thus calculated has a value corresponding to a value which depends on the surviving fraction $S'_i$ for each of the types of the cells that has been calculated with use of formula (3) or (4).

In formulation of a treatment plan, the values of the coefficient $\alpha_{new}$, the coefficient $\beta_{new}$, and the correction term C which correspond to the type of cells to be treated are applied to formula (3), an obtained surviving fraction $S'_i$ is applied to formula (5) to calculate a biological dose $d_{bio,i}$, and the calculated biological dose $d_{bio,i}$ is multiplied by the above given coefficient to calculate a clinical dose. Alternatively, the coefficients $\alpha_{new}$, $\beta_{new}$, $\gamma$, and $\delta$ which correspond to the type of cells to be treated are applied to formula (4), an obtained surviving fraction $S'_i$ is applied to formula (5) to calculate a biological dose $d_{bio,i}$, and the calculated biological dose $d_{bio,i}$ is multiplied by the above given coefficient to calculate a clinical dose. In the formulation of the treatment plan, when the degree of an effect on normal cells is evaluated, formula (3) or (4) is applied to the normal cells which are an evaluation target. Then, on the basis of the calculated clinical dose, a dose distribution in a case where an irradiation target is irradiated with radiation is estimated, and then the treatment plan for a patient is created. The creation of the treatment plan may include a step of creating a plan of irradiation of the irradiation target with the radiation, on the basis of the calculated clinical dose.

Note that an irradiation target irradiated with radiation is typically a human, but is not limited thereto, and may be an animal such as a dog and a cat. Specific examples of the target of irradiation with radiation include organs containing tumor regions.

In the conventional formulas, a dose is calculated only on the basis of an absorbed dose in standard cells and LET, without consideration of factors such as the type of cells, the type of radiation, and the dose (or the dose rate) of the radiation. Therefore, in a case where irradiation at an ultrahigh dose in which the FLASH effect is produced is carried out, a dose distribution calculated by the conventional method is likely to differ from a dose distribution of radiation that actually acts on cells which are an irradiation target. In particular, in the conventional formulas, it is not taken into consideration that, in a case where cells other than standard cells are irradiated with a heavy particle beam, a dose which actually acts on the cells that are an irradiation target varies depending on the type of the cells. Therefore, with the conventional formulas, it is impossible to accurately estimate a dose distribution in a case where an irradiation target containing cells that respond differently from standard cells is irradiated with a heavy particle beam.

In contrast, in formulas (3) and (4), which are formulas improved this time, and formula (5), in which these formulas are reflected, the fact that a response varies depending on the type of cells is taken into consideration. Specifically, the coefficient $\alpha_{new}$, the coefficient $\beta_{new}$, and the correction term C in formula (3) and the coefficients $\alpha_{new}$, $\beta_{new}$, $\gamma$, and $\delta$ in formula (4) are each a value that depends on the type of cells. In addition, in formulas (3) and (4) and formula (5), in which these formulas are reflected, an effect on a target in a case where irradiation is carried out at an ultrahigh dose is taken into consideration. Specifically, the correction term C in formula (3) and the coefficients $\gamma$ and $\delta$ in formula (4) are each a value that also depends on a dose (or a dose rate).

Thus, by employing a dose estimating method in which the improved formulas (3) and (4) are used, it is possible to more accurately formulate a treatment plan in treatment in which a heavy particle beam is used at an ultrahigh dose rate.

The dose estimating method in accordance with the present invention can also be used to optimize a dose distribution and a dose rate distribution in heavy particle radiotherapy at an ultrahigh dose rate. In heavy particle radiotherapy at an ultrahigh dose rate, a higher control rate with respect to a tumor and a lower adverse reaction incidence rate than in the past can be expected due to the synergistic effect of (i) the physical characteristic that a high dose of heavy particle beam concentrates on a tumor and (ii) the biological effectiveness that damage to a normal tissue is reduced due to the FLASH effect. Thus, an increase in opportunity for treating cancer in an aging society and a further improvement in QOL are expected.

Moreover, a reduction in adverse reaction or sequela certainly leads to a way toward a cancer or disease therapy with less burden to a body. In a case where an adverse reaction or a sequela occurs, temporal and economic burdens on a patient and a load on a medical resource for handling the adverse reaction or the sequela arise. However, by heavy particle radiotherapy at an ultrahigh dose rate being developed by the dose estimating method in accordance with the present invention, an economic effect brought about by reductions in these burdens can also be expected.

Although the method of calculating a clinical dose in accordance with the present invention has been described with reference to an example in which cells are irradiated with a heavy particle beam, the method of calculating a clinical dose in accordance with the present invention can also be applied to a case where cells are irradiated with a photon beam, an electron beam, or a proton beam.

(Specific example of calculation of surviving fraction in accordance with the present invention)

Fig. 5 shows survival curves obtained regarding HSG cells, which are often used as standard cells in heavy particle radiotherapy, in accordance with formula (4).

White circles (∘) and black circles (•) in Fig. 5 are data measured by an experiment of irradiation with a heavy particle beam. The white circles (∘) each indicate a result of irradiation with the heavy particle beam at a typical dose rate (0.1 Gy/s). The black circles (•) each indicate a result of irradiation with the heavy particle beam at an ultrahigh dose rate (100 Gy/s).

The coefficients $\alpha_{new}$, $\beta_{new}$, $\gamma$, and $\delta$ in formula (4) were determined so that results obtained from formula (4) best matched the results measured by the experiment of the irradiation with the heavy particle beam at a typical dose rate and an ultrahigh dose rate. Fig. 5 shows the survival curves obtained with use of formula (4). A bold solid line (reference sign 501) in Fig. 5 is a survival curve which was obtained in accordance with formula (4) and which was obtained at the ultrahigh dose rate. A thin solid line (reference sign 502) and a thin broken line (reference sign 503) in Fig. 5 are survival curves which were calculated, for comparison, in accordance with the conventional formula (1) and which were respectively obtained at the typical dose rate and the ultrahigh dose rate.

As shown in Fig. 5, it is obvious that estimation data on a surviving fraction $S'_i$ calculated by formula (4) of the present invention well matches results of measured surviving fractions in a wide range from a low dose range to a high dose range also at the ultrahigh dose rate. As has been described, it is possible to calculate a biological dose from the surviving fraction $S'_i$ and further calculate a clinical dose. Thus, it is clear that the present invention makes it possible to estimate a clinical dose more accurately than the conventional method.

(Configuration of dose evaluating device 1)

Next, the configuration of a dose evaluating device 1 which employs the dose evaluating method in accordance with the present invention so as to calculate a clinical dose is described with reference to Fig. 6. Fig. 6 is a block diagram illustrating an example of the configuration of the dose evaluating device 1 in accordance with an aspect of the present invention. The following description will discuss the configuration of the dose evaluating device 1 in accordance with an aspect of the present invention with reference to Fig. 6. The dose evaluating device 1 is a device to which a method of calculating a dose with use of formulas (3) and (5) or formulas (4) and (5), which were created by the inventors of the present application as has been described, and is capable of calculating a dose with use of inputted information and the improved formulas and outputting information that indicates a dose distribution based on a calculated value.

As illustrated in Fig. 6, the dose evaluating device 1 includes a control section 11, a storage section 12, and an output section 13. The control section 11 carries out a computation process and control of operation of each section of the dose evaluating device 1. The control section 11 includes a calculation information obtaining section 111, an absorbed dose obtaining section 112, a calculating section 113, and an evaluation information generating section 114.

The calculation information obtaining section 111 obtains calculation target information that is information necessary for a computation, and outputs the obtained information to the calculating section 113. The calculation target information includes (i) information that pertains to radiation with which an irradiation target is irradiated and (ii) information that pertains to the types of cells constituting the irradiation target which is irradiated with the radiation. The information that pertains to the radiation includes information that pertains to the dose or the dose rate of the radiation. The dose evaluating device 1 may include an input section that is not illustrated, and the calculation information obtaining section 111 may obtain the calculation target information in a case where various pieces of information are inputted into the input section by a user of the dose evaluating device 1.

The absorbed dose obtaining section 112 obtains, for each of the types of the cells constituting the irradiation target, absorbed dose information that is information necessary for the computation, and outputs the obtained information to the calculating section 113. The absorbed dose information is information that indicates, for each of the types of the cells constituting the irradiation target, an absorbed dose which is the energy of the radiation absorbed by cells of the each of the types. The dose evaluating device 1 may include an input section that is not illustrated, and the absorbed dose obtaining section 112 may obtain the absorbed dose information in a case where various pieces of information are inputted into the input section by a user of the dose evaluating device 1.

The calculating section 113 calculates a clinical dose for each of the types of the cells on the basis of the calculation target information and the absorbed dose information received from the calculation information obtaining section 111 and the absorbed dose obtaining section 112, respectively. Alternatively, the calculating section 113 calculates a clinical dose corresponding to the radiation with which the target is irradiated, on the basis of the calculation target information and the absorbed dose information received from the calculation information obtaining section 111 and the absorbed dose obtaining section 112, respectively.

Specifically, in a case where the calculating section 113 obtains the calculation target information and the absorbed dose information, which are necessary for the computation, from the calculation information obtaining section 111 and the absorbed dose obtaining section 112, respectively, the calculating section 113 refers to the storage section 12 and obtains formula data 121 used for the computation. In a case where the formula data 121 is data on the above-described formulas (3) and (5) or the above-described formulas (4) and (5), the calculating section 113 calculates a surviving fraction $S'_i$ for each of the types of the cells on the basis of the obtained information and the obtained formula (3) or (4). For example, on the basis of formula (3) or (4), the calculating section 113 calculates the surviving fraction $S'_i$ that depends on the type of the cells, the type of the radiation, the quality of the radiation, and the dose (or the dose rate) of the radiation. The calculating section 113 further calculates a biological dose $d_{bio,i}$ on the basis of the calculated surviving fraction $S'_i$ for each of the types of the cells and formula (5). Furthermore, the calculating section 113 calculates a clinical dose on the basis of the biological dose $d_{bio,i}$. The surviving fraction $S'_i$ and the biological dose $d_{bio,i}$ depend on the type of the cells. Therefore, it is possible for the calculating section 113 to calculate the clinical dose for each of the types of the cells. Note that the calculating section 113 may be configured to calculate the absorbed dose, the clinical dose, and the like with use of the method disclosed in Japanese Patent No. 5454989, on the basis of the surviving fraction $S'_i$ for each of the types of the cells.

The evaluation information generating section 114 creates evaluation information that indicates a dose distribution for each of the types of the cells, on the basis of the calculated clinical dose. The evaluation information generating section 114 controls the output section 13 to output the evaluation information. The evaluation information is information that indicates an evaluation on the radiation with which irradiation is carried out, and may be, for example, the surviving fraction $S'_i$ or the biological dose $d_{bio,i}$ each of which has been calculated by the calculating section 113 for each of the types of the cells. The evaluation information generating section 114 may further process any of the calculated values and cause the output section 13 to output, as the evaluation information, information that indicates a result of the processing. For example, the evaluation information generating section 114 may calculate the clinical dose from the biological dose $d_{bio,i}$ calculated by the calculating section 113, and cause the output section 13 to output the clinical dose as the evaluation information. The evaluation information generating section 114 may process the surviving fraction $S'_i$ or the biological dose $d_{bio,i}$ each of which has been calculated by the calculating section 113 for each of the types of the cells, and further cause the output section 13 to output the processed surviving fraction $S'_i$ or biological dose $d_{bio,i}$ in the form of a dose volume histogram (DVH), a dose rate volume histogram (DRVH), or the like.

The evaluation information generating section 114 may create, for example, a dose distribution map that indicates a dose distribution in the body of the target, on the basis of the clinical dose for each of the types of the cells. The evaluation information generating section 114 may then cause the output section 13 to output the evaluation information including the dose distribution map. For example, in a case where the calculating section 113 calculates biological doses $d_{bio,i}$ in cells (for example, organs) at a plurality of sites in the body of the target, the evaluation information generating section 114 may create a dose distribution map that is obtained by drawing, on image data obtained by capturing an image of the inside of the body of the target, contour lines corresponding to the levels of the biological doses or clinical doses calculated from the biological doses. The image data may be an X-ray computed tomography (CT) image, a magnetic resonance imaging (MRI) image, or the like. The imaging data may be alternatively a virtual CT image that is constructed from the Dual Energy CT or a virtual CT image that is constructed from an estimation model in which AI is used.

The storage section 12 is a storage device in which, for example, various computer programs that are read out by the control section 11 and data that are used in various processes carried out by the control section 11 are stored. For example, in the storage section 12, the formula data 121 on formulas (3) and (5) or formulas (4) and (5), which are used by the calculating section 113 for the computation, are stored. In the storage section 12, for example, a table may also be stored which is for converting, into parameters that can be applied to the computation carried out by the calculating section 113, various pieces of data obtained by the calculation information obtaining section 111 and the absorbed dose obtaining section 112. In addition, in a case where the evaluation information generating section 114 further carries out processing of data, for example, a computation and the like, data such as a formula or a table used for the computation and the like is stored in the storage section 12. In a case where the other computations are carried out, data on formulas for the computations is also stored.

The output section 13 is, for example, a display that can display data. In accordance with control by the evaluation information generating section 114, the output section 13 outputs the evaluation information such as the value calculated by the calculating section 113 or the data processed by the evaluation information generating section 114, in a form in which a user who uses the dose evaluating device 1 can confirm the evaluation information.

As has been described, the dose evaluating device 1 in accordance with the present invention is capable of (i) carrying out a computation with use of the new formulas as a computation for estimating a biological dose in heavy particle radiotherapy, (ii) visualizing a dose distribution in a body, and (iii) providing the dose distribution to a user such as a medical professional.

(Flow of processes carried out by dose evaluating device 1)

Next, a flow of processes carried out by the dose evaluating device 1 is described with reference to Fig. 7. Fig. 7 is a flowchart illustrating an example of the flow of the processes carried out in the dose evaluating device 1 (estimating method).

First, the calculation information obtaining section 111 obtains calculation target information that is information necessary for a computation carried out by the calculating section 113 (S1: calculation information obtaining step), and outputs the calculation target information to the calculating section 113. For example, the calculation information obtaining section 111 obtains the calculation target information by communication with an external device in which an input from a user into an input device or each piece of information is stored.

Next, the absorbed dose obtaining section 112 obtains absorbed dose information that indicates, for each of the types of cells constituting an irradiation target, an absorbed dose which is the energy of radiation absorbed by cells of the each of the types (S2: absorbed dose obtaining step), and outputs the absorbed dose information to the calculating section 113. For example, the absorbed dose obtaining section 112 obtains the absorbed dose information by communication with the external device in which the input from the user into the input device or each piece of information is stored.

In a case where the calculating section 113 obtains the calculation target information and the absorbed dose information from the calculation information obtaining section 111 and the absorbed dose obtaining section 112, respectively, the calculating section 113 refers to the storage section 12 and obtains formula data 131 used for the computation. The calculating section calculates a surviving fraction $S'_i$ for each of the types of the cells on the basis of the obtained evaluation target information and the obtained absorbed dose information with use of formula (3) or (4) among the formulas (S3).

In a case where the calculating section 113 calculates the surviving fraction $S'_i$, the calculating section 113 calculates a biological dose $d_{bio,i}$ for each of the types of the cells on the basis of the surviving fraction $S'_i$ with use of formula (5). The calculating section 113 outputs, to the evaluation information generating section 114, information that indicates the calculated biological dose $d_{bio,i}$. The calculating section 113 further calculates, for each of the types of the cells, a clinical dose for the each of the types of the cells with use of the biological dose $d_{bio,i}$ calculated on the basis of the calculation target information. In other words, the calculating section 113 calculates, for each of the types of the cells, a clinical dose of the radiation with which the irradiation target is irradiated, on the basis of the calculation target information and the absorbed dose information (S4: calculating step). Specifically, the calculating section 113 rescales the calculated biological dose $d_{bio,i}$ by multiplying the biological dose $d_{bio,i}$ by an empirical coefficient (for example, 1.46) for converting a cell response to a tissue response, thereby calculating the clinical dose.

The evaluation information generating section 114 carries out a further computation and data processing with respect to the calculated biological dose $d_{bio,i}$ or the calculated clinical dose so as to create a dose distribution map or the like. Thereafter, the evaluation information generating section 114 controls the output section 13 to output the dose calculated by the calculating section 113 or evaluation information created on the basis of the dose, for example, the clinical dose (S5).

(Effects)

The dose evaluating device 1 in accordance with the present invention uses the method of calculating a dose with use of the above-described formulas (3) and (5) or the above-described formulas (3) and (5). This makes it possible to more accurately formulate a treatment plan in treatment in which a heavy particle beam is used at an ultrahigh dose rate. Moreover, the dose evaluating device in accordance with the present invention makes it possible to visualize a dose distribution on the basis of a calculated dose and provide the dose distribution to a medical professional.

Embodiment 2

The functions of the dose evaluating device 1 can be realized by a program for causing a computer to function as the device, the program causing the computer to function as control blocks (in particular, the sections included in the control section 11) of the dose evaluating device.

In this case, the dose evaluating device includes, as hardware for executing the program, a computer that includes at least one control device (for example, processor) and at least one storage device (for example, memory). By the control device executing the program with reference to the storage device, the functions described in the above embodiment are realized.

The program may be recorded in one or more non-transitory computer-readable recording media. The one or more recording media may or may not be included in the dose evaluating device. In the latter case, the program may be made available to the dose evaluating device via any wired or wireless transmission medium.

Some or all of the functions of the control blocks can also be realized by a logic circuit. For example, the present invention also encompasses, in its scope, an integrated circuit in which a logic circuit that functions the control blocks is formed. In another example, the functions of the control blocks can also be realized by a quantum computer.

The processes described in the above embodiment may be executed by artificial intelligence (AI). In this case, the AI may operate in the control device, or may operate in another device (for example, an edge computer or a cloud server).

Aspects of the present invention can also be expressed as follows:

As has been described, a dose evaluating method in accordance with a first aspect of the present invention includes: a calculation information obtaining step of obtaining calculation target information including (i) information that pertains to radiation with which an irradiation target is irradiated and that includes a dose or a dose rate of the radiation and (ii) information that pertains to types of cells constituting the irradiation target which is irradiated with the radiation; an absorbed dose obtaining step of obtaining absorbed dose information that indicates, for each of the types of the cells constituting the irradiation target, an absorbed dose which is energy of the radiation absorbed by cells of the each of the types; and a calculating step of calculating, for each of the types of the cells, a clinical dose of the radiation with which the irradiation target is irradiated, on the basis of the calculation target information and the absorbed dose information.

In a case where a target is irradiated with radiation, an absorbed dose which is energy of the radiation absorbed by the target may vary for each of the types of cells. Furthermore, the absorbed dose may vary depending on the dose rate of the radiation with which the target is irradiated. According to the above configuration, it is possible to calculate a clinical dose for each of the types of the cells, on the basis of (i) calculation target information including information that pertains to the radiation and information that pertains to the types of the cells and (ii) absorbed dose information for each of the types of the cells constituting the irradiation target.

The dose evaluating method in accordance with a second aspect of the present invention may be configured such that, in the first aspect, the information that pertains to the radiation includes information that pertains to a type and a quality of the radiation, and in the calculating step, a surviving fraction $S'_i$ is calculated by formula (I) below for the each of the types of the cells, and, on the basis of the surviving fraction $S'_i$ which has been calculated, the clinical dose is calculated for the each of the types of the cells constituting the irradiation target:

$$S'_i = exp\left(-\alpha_{new}d_i - \beta_{new}d_i^2\right) + C \quad \cdots \text{formula (I)}$$

where

d$_i$ is the absorbed dose for the each of the types of the cells,
a coefficient $\alpha_{new}$ and a coefficient $\beta_{new}$ are each a value obtained, for the each of the types of the cells, from a survival curve that indicates a relationship between a surviving fraction and the dose of the radiation with which the irradiation target is irradiated and which has the type and the quality, and
a correction term C is a value for correcting, in accordance with the dose or the dose rate of the radiation, a surviving fraction obtained with use of the coefficient $\alpha_{new}$ and the coefficient $\beta_{new}$.

According to the above configuration, it is possible to more accurately evaluate a clinical dose on the basis of formula (I), which is a formula improved over a formula used conventionally.

The dose evaluating method in accordance with a third aspect of the present invention may be configured such that, in the first aspect, the information that pertains to the radiation includes information that pertains to a type and a quality of the radiation, and in the calculating step, a surviving fraction S'$_i$ is calculated by formula (II) below for the each of the types of the cells, and, on the basis of the surviving fraction S'$_i$ which has been calculated, the clinical dose is calculated for the each of the types of the cells constituting the irradiation target:

$$S'_i = exp\left(-\alpha_{new}d_i - \beta_{new}d_i^2 \frac{1}{1 + \frac{\beta_{new}}{\gamma}d_i + \delta d_i^2}\right) \cdots \text{formula (II)}$$

where

d$_i$ is the absorbed dose for the each of the types of the cells,
a coefficient $\alpha_{new}$, a coefficient $\beta_{new}$, a coefficient y, and a coefficient $\delta$ are each a value obtained, for the each of the types of the cells, from a survival curve that indicates a relationship between a surviving fraction and the dose of the radiation with which the irradiation target is irradiated and which has the type and the quality, and
the coefficient $\gamma$ and the coefficient $\delta$ are each the value which depends on the dose or the dose rate of the radiation.

According to the above configuration, it is possible to more accurately evaluate a clinical dose on the basis of formula (II), which is a formula improved over the formula used conventionally.

The dose evaluating method in accordance with a fourth aspect of the present invention may be configured such that, in any one of the first through third aspects, the radiation is one of a heavy particle beam, a photon beam, an electron beam, and a proton beam.

According to the above configuration, it is possible to calculate a more accurate clinical dose. Therefore, it is possible to more accurately calculate a clinical dose in radiotherapy in which a heavy particle beam, an electron beam, a proton beam, or the like is used. Therefore, by using the present dose evaluating method, it is possible to create an appropriate and safe treatment plan for radiotherapy.

The dose evaluating method in accordance with a fifth aspect of the present invention may be configured such that, in any one of the first through third aspects, the radiation is a heavy particle beam, and the dose rate of the radiation is in an ultrahigh dose rate range.

According to the above configuration, a clinical dose that is more accurate than a conventional one is calculated. Therefore, it is possible to more accurately formulate a treatment plan in treatment in which a heavy particle beam is used at an ultrahigh dose rate.

The dose evaluating method in accordance with a sixth aspect of the present invention may be configured such that, in the fifth aspect, the dose rate of the radiation is not less than 40 Gy/second.

An irradiation planning method in accordance with a seventh aspect of the present invention is a method of creating a plan of irradiation of an irradiation target with radiation, on the basis of a clinical dose calculated by the dose evaluating method in any one of the first through third aspects.

According to the above configuration, a plan of irradiation is created on the basis of a more accurate clinical dose in which the type of cells and a dose rate are taken into consideration. Therefore, it is possible to create a more appropriate and safer plan of irradiation in radiotherapy.

A dose evaluating device in accordance with an eighth aspect of the present invention includes: a calculation information obtaining section which obtains calculation target information including (i) information that pertains to radiation with which an irradiation target is irradiated and that includes a dose or a dose rate of the radiation and (ii) information that pertains to types of cells constituting the irradiation target which is irradiated with the radiation; an absorbed dose obtaining section which obtains absorbed dose information that indicates, for each of the types of the cells constituting the irradiation target, an absorbed dose which is energy of the radiation absorbed by cells of the each of the types; and a calculating section which calculates, for each of the types of the cells, a clinical dose of the radiation with which the irradiation target is irradiated, on the basis of the calculation target information and the absorbed dose information.

According to the above configuration, an effect similar to that brought about in the first aspect is brought about.

A control program in accordance with a ninth aspect of the present invention is a control program for causing a computer to function as the dose evaluating device described in the eighth aspect, the control program causing the computer to function as the calculating section.

A recording medium in accordance with a tenth aspect of the present invention is a computer-readable recording medium in which the control program described in the ninth aspect is recorded.

The present invention is not limited to the embodiments, but can be altered by a skilled person in the art within the scope of the claims. The present invention also encompasses, in its technical scope, any embodiment derived by combining technical means disclosed in differing embodiments.

Reference Signs List

| 1 | Dose evaluating device |
|---|---|
| 111 | Calculation information obtaining section |
| 112 | Absorbed dose obtaining section |
| 113 | Calculating section |
| 114 | Evaluation information generating section |
| S1 | Calculation information obtaining step |
| S2 | Absorbed dose obtaining step |
| S4 | Calculating step |

**Claims**

1. A dose evaluating method comprising:

   a calculation information obtaining step of obtaining calculation target information including (i) information that pertains to radiation with which an irradiation target is irradiated and that includes a dose or a dose rate of the radiation and (ii) information that pertains to types of cells constituting the irradiation target which is irradiated with the radiation;
   an absorbed dose obtaining step of obtaining absorbed dose information that indicates, for each of the types of the cells constituting the irradiation target, an absorbed dose which is energy of the radiation absorbed by cells of the each of the types; and
   a calculating step of calculating, for each of the types of the cells, a clinical dose of the radiation with which the irradiation target is irradiated, on the basis of the calculation target information and the absorbed dose information.

2. The dose evaluating method as set forth in claim 1, wherein

   the information that pertains to the radiation includes information that pertains to a type and a quality of the radiation, and
   in the calculating step, a surviving fraction $S'_i$ is calculated by formula (I) below for the each of the types of the cells, and, on the basis of the surviving fraction $S'_i$ which has been calculated, the clinical dose is calculated for the each of the types of the cells constituting the irradiation target:

$$S'_i = exp\left(-\alpha_{new}d_i - \beta_{new}d_i^2\right) + C \quad \cdots \text{formula (I)}$$

   where

$d_i$ is the absorbed dose for the each of the types of the cells,
a coefficient $\alpha_{new}$ and a coefficient $\beta_{new}$ are each a value obtained, for the each of the types of the cells, from a survival curve that indicates a relationship between a surviving fraction and the dose of the radiation with which the irradiation target is irradiated and which has the type and the quality, and
a correction term C is a value for correcting, in accordance with the dose or the dose rate of the radiation, a surviving fraction obtained with use of the coefficient $\alpha_{new}$ and the coefficient $\beta_{new}$.

3. The dose evaluating method as set forth in claim 1, wherein

the information that pertains to the radiation includes information that pertains to a type and a quality of the radiation, and
in the calculating step, a surviving fraction $S'_i$ is calculated by formula (II) below for the each of the types of the cells, and, on the basis of the surviving fraction $S'_i$ which has been calculated, the clinical dose is calculated for the each of the types of the cells constituting the irradiation target:

$$S'_i = exp\left(-\alpha_{new}d_i - \beta_{new}d_i^2 \frac{1}{1 + \frac{\beta_{new}}{\gamma}d_i + \delta d_i^2}\right) \cdots \text{formula (II)}$$

where
$d_i$ is the absorbed dose for the each of the types of the cells,
a coefficient $\alpha_{new}$, a coefficient $\beta_{new}$, a coefficient y, and a coefficient $\delta$ are each a value obtained, for the each of the types of the cells, from a survival curve that indicates a relationship between a surviving fraction and the dose of the radiation with which the irradiation target is irradiated and which has the type and the quality, and
the coefficient y and the coefficient $\delta$ are each the value which depends on the dose or the dose rate of the radiation.

4. The dose evaluating method as set forth in any one of claims 1 through 3, wherein the radiation is one of a heavy particle beam, a photon beam, an electron beam, and a proton beam.

5. The dose evaluating method as set forth in any one of claims 1 through 3, wherein the radiation is a heavy particle beam, and the dose rate of the radiation is in an ultrahigh dose rate range.

6. The dose evaluating method as set forth in claim 5, wherein the dose rate of the radiation is not less than 40 Gy/ second.

7. An irradiation planning method of creating a plan of irradiation of an irradiation target with radiation, on the basis of a clinical dose calculated by the dose evaluating method recited in any one of claims 1 through 3.

8. A dose evaluating device comprising:

a calculation information obtaining section which obtains calculation target information including (i) information that pertains to radiation with which an irradiation target is irradiated and that includes a dose or a dose rate of the radiation and (ii) information that pertains to types of cells constituting the irradiation target which is irradiated with the radiation;
an absorbed dose obtaining section which obtains absorbed dose information that indicates, for each of the types of the cells constituting the irradiation target, an absorbed dose which is energy of the radiation absorbed by cells of the each of the types; and
a calculating section which calculates, for each of the types of the cells, a clinical dose of the radiation with which the irradiation target is irradiated, on the basis of the calculation target information and the absorbed dose information.

9. A control program for causing a computer to function as the dose evaluating device recited in claim 8, the control program causing the computer to function as the calculation information obtaining section, the absorbed dose obtaining section, and the calculating section.

**10.** A computer-readable recording medium in which the control program recited in claim 9 is recorded.

## FIG. 1

## FIG. 2

## FIG. 3

## FIG. 4

FIG. 5

# FIG. 6

# FIG. 7

FIG. 8

FIG. 9

FIG. 10

FIG. 11

# EP 4 628 160 A1

## INTERNATIONAL SEARCH REPORT

| International application No. |
|---|
| **PCT/JP2023/041401** |

### A. CLASSIFICATION OF SUBJECT MATTER

***A61N 5/10***(2006.01)i
FI: A61N5/10 P; A61N5/10 H

According to International Patent Classification (IPC) or to both national classification and IPC

### B. FIELDS SEARCHED

Minimum documentation searched (classification system followed by classification symbols)

A61N

Documentation searched other than minimum documentation to the extent that such documents are included in the fields searched

Published examined utility model applications of Japan 1922-1996
Published unexamined utility model applications of Japan 1971-2024
Registered utility model specifications of Japan 1996-2024
Published registered utility model applications of Japan 1994-2024

Electronic data base consulted during the international search (name of data base and, where practicable, search terms used)

### C. DOCUMENTS CONSIDERED TO BE RELEVANT

| Category* | Citation of document, with indication, where appropriate, of the relevant passages | Relevant to claim No. |
|---|---|---|
| X | JP 2009-160308 A (TOSHIBA CORP.) 23 July 2009 (2009-07-23)<br>paragraphs [0087]-[0186] | 1-10 |
| A | JP 2013-248133 A (JAPAN ATOMIC ENERGY AGCY.) 12 December 2013 (2013-12-12)<br>paragraphs [0066]-[0082] | 1-10 |
| A | JP 2022-154269 A (HITACHI, LTD.) 13 October 2022 (2022-10-13)<br>paragraphs [0059]-[0076] | 1-10 |
| A | JP 2018-108284 A (TOSHIBA CORP.) 12 July 2018 (2018-07-12)<br>paragraphs [0080]-[0083] | 1-10 |
| A | JP 2022-166206 A (VIEWRAY TECHNOLOGIES, INC.) 01 November 2022 (2022-11-01)<br>paragraph [0026] | 1-10 |
| A | JP 2003-070921 A (MITSUBISHI ELECTRIC CORP.) 11 March 2003 (2003-03-11)<br>paragraphs [0016]-[0026] | 1-10 |

☑ Further documents are listed in the continuation of Box C.      ☑ See patent family annex.

| | |
|---|---|
| * Special categories of cited documents:<br>"A" document defining the general state of the art which is not considered to be of particular relevance<br>"D" document cited by the applicant in the international application<br>"E" earlier application or patent but published on or after the international filing date<br>"L" document which may throw doubts on priority claim(s) or which is cited to establish the publication date of another citation or other special reason (as specified)<br>"O" document referring to an oral disclosure, use, exhibition or other means<br>"P" document published prior to the international filing date but later than the priority date claimed | "T" later document published after the international filing date or priority date and not in conflict with the application but cited to understand the principle or theory underlying the invention<br>"X" document of particular relevance; the claimed invention cannot be considered novel or cannot be considered to involve an inventive step when the document is taken alone<br>"Y" document of particular relevance; the claimed invention cannot be considered to involve an inventive step when the document is combined with one or more other such documents, such combination being obvious to a person skilled in the art<br>"&" document member of the same patent family |

| Date of the actual completion of the international search | Date of mailing of the international search report |
|---|---|
| **12 January 2024** | **23 January 2024** |

| Name and mailing address of the ISA/JP | Authorized officer |
|---|---|
| **Japan Patent Office (ISA/JP)**<br>**3-4-3 Kasumigaseki, Chiyoda-ku, Tokyo 100-8915**<br>**Japan** | |
| | Telephone No. |

Form PCT/ISA/210 (second sheet) (July 2022)

23

**INTERNATIONAL SEARCH REPORT**

International application No.

**PCT/JP2023/041401**

| C. | DOCUMENTS CONSIDERED TO BE RELEVANT | |
|---|---|---|
| Category* | Citation of document, with indication, where appropriate, of the relevant passages | Relevant to claim No. |
| A | JP 2019-514510 A (KONINKLIJKE PHILIPS N.V.) 06 June 2019 (2019-06-06)<br>paragraph [0025] | 1-10 |

Form PCT/ISA/210 (second sheet) (July 2022)

# EP 4 628 160 A1

## INTERNATIONAL SEARCH REPORT
### Information on patent family members

International application No.

**PCT/JP2023/041401**

| Patent document cited in search report | | | Publication date (day/month/year) | Patent family member(s) | | | Publication date (day/month/year) |
|---|---|---|---|---|---|---|---|
| JP | 2009-160308 | A | 23 July 2009 | US | 2009/0175418 | A1 | |
| | | | | paragraphs [0198]-[0361] | | | |
| | | | | EP | 2078537 | B1 | |
| JP | 2013-248133 | A | 12 December 2013 | (Family: none) | | | |
| JP | 2022-154269 | A | 13 October 2022 | US | 2022/0314027 | A1 | |
| | | | | paragraphs [0069]-[0096] | | | |
| JP | 2018-108284 | A | 12 July 2018 | (Family: none) | | | |
| JP | 2022-166206 | A | 01 November 2022 | US | 2019/0168028 | A1 | |
| | | | | paragraph [0026] | | | |
| | | | | WO | 2019/112880 | A1 | |
| | | | | EP | 3710112 | A1 | |
| | | | | CN | 111712298 | B | |
| JP | 2003-070921 | A | 11 March 2003 | (Family: none) | | | |
| JP | 2019-514510 | A | 06 June 2019 | US | 2019/0126072 | A1 | |
| | | | | paragraph [0029] | | | |
| | | | | WO | 2017/186522 | A1 | |
| | | | | EP | 3449269 | A1 | |
| | | | | CN | 109073721 | B | |

Form PCT/ISA/210 (patent family annex) (July 2022)

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- JP 5454989 B

- JP 2019180908 A